# EUROPÄISCHE PATENTSCHRIFT

(11) **EP 0 961 592 B1**
(45) Veröffentlichungstag und Bekanntmachung des Hinweises auf die Patenterteilung: **24.09.2003**
(21) Anmeldenummer: 98907890.2
(22) Anmeldetag: 04.02.1998
(51) Int. Cl.: A61C 8/00, A61C 13/30

(54) **IMPLANTAT, INSBESONDERE ZAHNWURZELIMPLANTAT**
IMPLANT, SPECIALLY A TOOTH ROOT IMPLANT
IMPLANT, NOTAMMENT IMPLANT DE RACINE DE DENTS

(30) Priorität: 11.02.1997 DE 19707310
(43) Veröffentlichungstag der Anmeldung: 08.12.1999
(73) Patentinhaber: raro plastics gmbh, 12099 Berlin (DE)
(72) Erfinder: BARUSCHKE, Michael, D-14195 Berlin (DE); KÄUFER, Helmut, D-40822 Mettmann (DE); BONGERS, Alexander, D-66606 St. Wendel (DE)
(74) Vertreter: Böning, Manfred, Prof.Dr.-Ing.
(86) Internationale Anmeldenummer: DE9800395
(87) Internationale Veröffentlichungsnummer: WO98034560

(56) Entgegenhaltungen:
- WO-A-95/13028
- US-A- 3 934 347
- US-A- 4 244 689
- US-A- 4 252 525
- US-A- 4 812 120

## Beschreibung

### Technisches Gebiet

Die Erfindung betrifft ein Implantat, insbesondere Zahnwurzelimplantat, mit einem Grundkörper, der als elastischer Kunststoffkörper ausgebildet ist.

### Stand der Technik

Wie von Fallschlüssel in "Zahnärztliche Implantologie", Quintessenz Verlags-GmbH, Berlin u.a., 1986, S. 94-95 beschrieben, werden in der Medizin zahlreiche Kunststoffe zu Implantationszwecken verwendet. Aus der US 45 31 916 ist außerdem bekannt, Zahnwurzelimplantate aus unterschiedlichen Materialien mit einem porösen Kunststoffmantel zu versehen, um das Einwachsen von Körpergewebe in die Oberfläche des Implantates zu erleichtern. Kunststoffimplantate bieten im zahnärztlichen Bereich den Vorteil, daß sie dem Patienten ein gegenüber Metallimplantaten gewohnteres "Kaugefühl" vermitteln. Der Grund hierfür dürfte darin bestehen, daß die Struktur von Kunststoffen weniger stark von der Struktur des Kieferknochengewebes abweicht als eine Metallstruktur. Ein Problem, das sich in besonders starkem Maße bei Metallimplantaten, aber auch bei Kunststoffimplantaten noch nicht befriedigend gelöst ist, besteht darin, daß die Einwachszeiten der Implantate vergleichsweise lang sind. Insbesondere bei Metallimplantaten sind Einwachszeiten von bis zu sechs Monaten nicht ungewöhnlich.

Um das Einwachsen eines Implantates zu verbessern, ist in der US 3 934 347 bereits vorgeschlagen worden, einen mit einem Flansch und einem an einem Ende mit einem Boden versehenen Metallzylinder mit einem in eine Silikonmasse eingebetteten Keramik- oder Metallring zu umgeben und auf bestimmte Abschnitte der Silikonmasse faseriges oder gewebtes Material zu applizieren, indem man die Fasern entweder in das Silikon einbettet oder aber auf die Silikonoberfläche klebt.

### Darstellung der Erfindung

Der Erfindung liegt die Aufgabe zugrunde, ein Implantat zu schaffen, das insbesondere bei Anwendung im Bereich der zahnärztlichen Praxis eine deutliche Verkürzung der Einwachszeiten ermöglicht und darüber hinaus die Gewähr für eine bessere Homogenität von Implantat und Implantathaltebereich bietet. Diese Aufgabe wird bei einem Implantat der eingangs beschriebenen Art erfindungsgemäß dadurch gelöst, daß mindestens ein Teil der Außenfläche des Grundkörpers aufgefasert ist, daß die Fasern der aufgefaserten Außenfläche im grundkörpernahen Bereich jeweils eine Übergangszone mit sich zum Grundkörper erweiterndem Querschnitt aufweisen und daß die Festigkeit des Kunststoffes im Bereich der Fasern größer als im übrigen Bereich des Grundkörpers ist.

Das erfindungsgemäße Implantat bietet den Vorteil, daß sich die faserartig strukturierte Außenfläche des Implantates gewissermaßen tentakelartig mit dem Körpergewebe verbindet. Auf diese Weise werden nicht nur die Einwachszeiten spürbar verkürzt, sondern man erhält darüber hinaus eine Übergangszone zwischen Kunststoffimplantat und Körpergewebe, die sowohl bei schlagartigen Belastungen des Implantates als auch beim normalen Kauvorgang eine Pufferfunktion erfüllt.

Als besonders vorteilhaft erweist es sich, wenn der Grundkörper des Implantates mit einer Bohrung und mit sich über einen Teil der Grundkörperlänge erstreckenden, ein Aufweiten des Grundkörpers erleichternden Schlitzen versehen ist. Das Aufweiten des Grundkörpers durch Einführen entsprechend dimensionierter Schrauben in die Bohrung des Grundkörpers bietet im Dentalbereich die Möglichkeit, den Durchmesser des beim Einsetzen des Implantates aufgeweiteten Grundkörpers im Verlauf der Einwachsphase mehr oder weniger kontinuierlich zu verringern und so die Größe und die Struktur der oben erwähnten Pufferzone zu beeinflussen.

### Kurze Beschreibung der Zeichnungen

Es zeigen:
Fig. 1 ein als Vollkörper ausgebildetes Zahnwurzelimplantat,
Fig. 2 ein als Hohlkörper mit Innengewinde ausgebildetes Zahnwurzelimplantat,
Fig. 3 ein als mit Längsschlitzen versehener Hohlkörper ausgebildetes Zahnwurzelimplantat,
Fig. 4 einen Schnitt längs der Linie IV-IV in Fig. 3,
Fig. 5 einen Schnitt längs der Linie V-V in Fig. 4,
Fig. 6 einen Oberflächenausschnitt eines modifizierten Implantates und
Fig. 7 in stark vergrößertem Maßstab die über die Außenfläche des Grundkörpers eines Implantates vorstehenden Fasern.

### Wege zur Ausführung der Erfindung

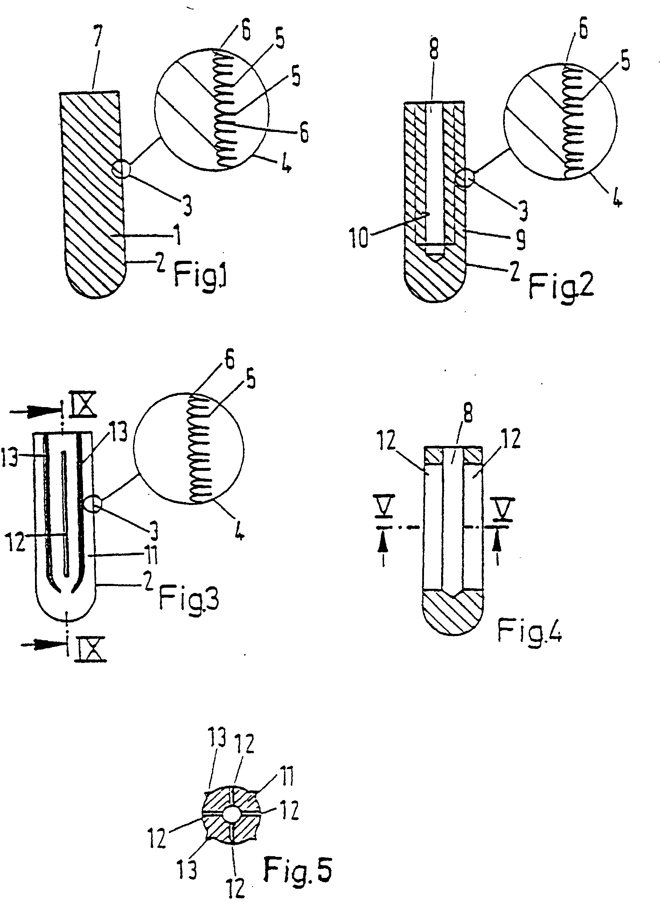

In Figur 1 ist 1 der aus einem biokompatiblen Kunststoff bestehende Grundkörper eines Zahnwurzelimplantates. Die zylindrische Außenfläche 2 des Grundkörpers 1 ist nicht glatt, sie hat vielmehr eine als aufgefasert bezeichenbare Mikrostruktur, wie dies in vergrößertem Maßstab im dem Ausschnitt 3 des Grundkörpers 1 entsprechenden Kreis 4 angedeutet ist. Man erkennt eine Vielzahl von Fasern 5, die im Bereich ihres Überganges in den Grundkörper 1 eine Zone 6 aufweisen, in der ihr Querschnitt zum Grundkörper hin zunimmt. Die Fasern 5 werden mit anderen Worten von sich verjüngenden, unregelmäßigen Mikrovorsprüngen der Außenfläche 2 des Grundkörpers gebildet. Während der größere Teil der Außenfläche des Grundkörpers 1 aufgefasert, d.h. mit gereckten Mikrofasern und/oder Mikrohohlfasern unregelmäßiger Form und Größe versehen ist, bleibt dessen Stirnfläche 7 glatt. Die glatte Stirnfläche 7 läßt sich nutzen, um mit dem Zahnwurzelimplantat durch Spiegelschweißen ein Kunststoffteil zu verbinden, indem letzeres durch eine Reibbewegung mit der Stirnfläche 7 verschmolzen wird.

Die Figur 2 zeigt einen mit einer zentralen Bohrung 8 versehenen Grundkörper 9, dessen zylindrische Außenfläche 2 wie im Falle des Grundkörpers 1 wiederum aufgefasert ist. Die Bohrung 8 ist mit einem Innengewinde 10 zur Aufnahme nicht dargestellter Schrauben versehen. Durch die Wahl eines geeigneten Schraubendurchmessers läßt sich das Implantat in leicht aufgeweitetem Zustand in die zu seiner Aufnahme bestimmte Bohrung im Kiefer eines Patienten einsetzen.

Bei dem in den Figuren 3 bis 5 dargestellten Zahnwurzelimplantat weist der Grundkörper 11 außer einer zentralen Bohrung 8 vier über seinen Umfang verteilte, ein Aufspreizen erleichternde Schlitze 12 und vier zur Zentrierung und Rotationssicherung dienende Längsrippen 13 auf.

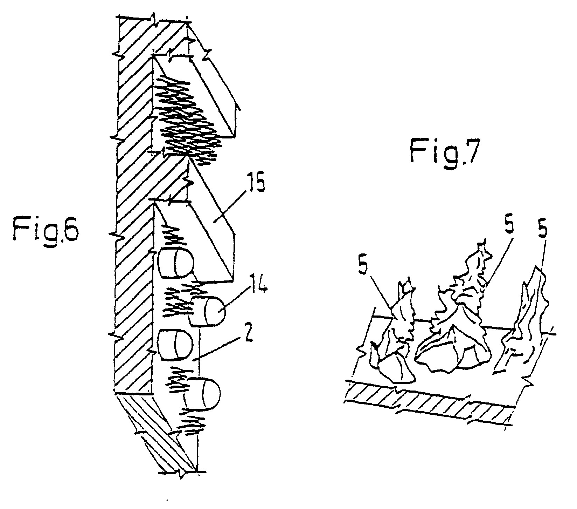

Um ein Flachdrücken der Fasern 5 beim Einsetzen in eine hierfür vorbereitete Aufnahme im Kieferknochen eines Patienten zu verhindern, kann es zweckmäßig sein, wie in Figur 6 dargestellt, die zylindrische Außenfläche 2 des jeweiligen Grundkörpers zusätzlich mit warzen- und/oder stegförmigen Distanzhaltern 14 bzw. 15 zu versehen. Die Zwischenräume zwischen den Distanzhaltern 14 und 15 lassen sich im übrigen zur Anbringung eines Überzuges aus einer das Knochenwachstum anregenden Substanz, vorzugsweise in Form von Kollagenen nutzen.

Figur 7 zeigt schließlich, daß es sich bei den Fasern 5 nicht um mehr oder weniger glatte und einen sich kontinuierlich verjüngenden Querschnitt aufweisende Gebilde handelt, sondern daß sie eine stark zerklüftete, lamellen- bzw. lappenförmige Auswüchse aufweisende Struktur haben, die die Ansiedlung von Körpergewebezellen begünstigt. Die Länge der Fasern 5 kann ungleich groß sein, sie beträgt bei Zahnimplantaten maximal 1 mm. Durch Verändern der Länge und der Mikrostruktur der Fasern 5 ist es möglich, das Implantat an die Struktur des zu seiner Aufnahme bestimmten Knochens anzupassen.

Dadurch, daß die Fasern 5 im Zuge der Herstellung der Implantate gewissermaßen unter Aufbrechen ihrer Außenfläche aus dem jeweiligen Grundkörper extrahiert werden und es dabei zu einer Streckung ihrer Molekülstrukturen kommt, stellt sich ein erwünschter Effekt ein, der darin besteht, daß es zu einer Steigerung des E-Moduls kommt, die insbesondere zur Erhöhung der Zugfestigkeit des Fasermaterials gegenüber der Zugfestigkeit des Kunststoffes des Grundkörpers führt.

In den Figuren sind Implantate mit einem im wesentlichen zylindrischen Grundkörper dargestellt. Im Falle der Anwendung sogenannter Direktimplantate kann dem Grundkörper stattdessen aber auch die Form der jeweils extrahierten Zahnwurzel gegeben werden.

## Patentansprüche

1. Implantat, insbesondere Zahnwurzelimplantat, mit einem Grundkörper (1;9;11), der als elastischer Kunststoffkörper ausgebildet ist, **dadurch gekennzeichnet, daß** mindestens ein Teil der Außenfläche (2) des Grundkörpers (1;9;11) aufgefasert ist, daß die Fasern (5) der aufgefaserten Außenfläche (2) im grundkörpernahen Bereich jeweils eine Übergangszone (6) mit sich zum Grundkörper (1;9;11) erweiterndem Querschnitt aufweisen und daß die Festigkeit des Kunststoffes im Bereich der Fasern (5) größer als im übrigen Bereich des Grundkörpers (1;9;11) ist.

2. Implantat nach Anspruch 1, **dadurch gekennzeichnet, daß** der Grundkörper (9;11) mit einer zentralen Bohrung (8) versehen ist.

3. Implantat nach Anspruch 1 oder 2, **dadurch gekennzeichnet, daß** der Grundkörper (11) mit sich über einen Teil seiner Länge erstreckenden Schlitzen (12) versehen ist.

4. Implantat nach Anspruch 2 oder 3, **dadurch gekennzeichnet, daß** die Bohrung (8) mit einem Innengewinde (10) versehen ist.

5. Implantat nach einem oder mehreren der Ansprüche 1 bis 4, **dadurch gekennzeichnet, daß** die Außenfläche (2) des Grundkörpers mit von Vorsprüngen des Grundkörpers gebildeten Distanzhaltern (14,15) versehen ist (Fig. 6).

6. Implantat nach Anspruch 5, **dadurch gekennzeichnet, daß** die Distanzhalter (14) warzenförmig ausgebildet sind.

7. Implantat nach Anspruch 5, **dadurch gekennzeichnet, daß** die Distanzhalter (15) stegförmig ausgebildet sind.

8. Implantat nach einem oder mehreren der Ansprüche 1 bis 7, **dadurch gekennzeichnet, daß** die Außenfläche (2) des Grundkörpers (11) mit Längsrippen (13) versehen ist.

9. Implantat nach einem oder mehreren der Ansprüche 1 bis 8, **dadurch gekennzeichnet, daß** die Länge der Fasern (5) ungleich groß ist.

10. Implantat nach einem oder mehreren der Ansprüche 1 bis 9, **dadurch gekennzeichnet, daß** die Fasern (5) eine zerklüftete Oberflächenstruktur aufweisen.

11. Implantat nach einem oder mehreren der Ansprüche 1 bis 10, **dadurch gekennzeichnet, daß** die Außenfläche (2) des Grundkörpers (1;9;11) mit einem das Knochenwachstum anregenden Überzug versehen ist.

12. Implantat nach einem oder mehreren der Ansprüche 1 bis 11, **dadurch gekennzeichnet, daß** der Grundkörper eine an die Form einer extrahierten Zahnwurzel angepaßte Kontur aufweist.

## Claims

1. Implant, in particular tooth root implant, with a main body (1; 9; 11) which is designed as an elastic body made of plastic, **characterized in that** at least part of the outer surface (2) of the main body (1; 9; 11) is fibred, **in that** the fibres (5) of the fibred outer surface (2) have, in the area near the main body, in each case a transition zone (6) with a cross-section widening towards the main body (1; 9; 11), and **in that** the strength of the plastic in the area of the fibres (5) is greater than in the remainder of the main body (1; 9; 11).

2. Implant according to Claim 1, **characterized in that** the main body (9; 11) is provided with a central bore (8).

3. Implant according to Claim 1 or 2, **characterized in that** the main body (11) is provided with slots (12) extending over part of its length.

4. Implant according to Claim 2 or 3, **characterized in that** the bore (8) is provided with an internal thread (10).

5. Implant according to one or more of Claims 1 to 4, **characterized in that** the outer surface (2) of the main body is provided with spacer elements (14, 15) formed by projections of the main body.

6. Implant according to Claim 5, **characterized in that** the spacer elements (14) are of nipple-shaped design.

7. Implant according to Claim 5, **characterized in that** the spacer elements (15) are of ridge-shaped design.

8. Implant according to one or more of Claims 1 to 7, **characterized in that** the outer surface (2) of the main body (11) is provided with longitudinal ribs (13).

9. Implant according to one or more of Claims 1 to 8, **characterized in that** the fibres (5) are of unequal length.

10. Implant according to one or more of Claims 1 to 9, **characterized in that** the fibres (5) have a rugged surface structure.

11. Implant according to one or more of Claims 1 to 10, **characterized in that** the outer surface (2) of the main body (1; 9; 11) is provided with a coating which promotes bone growth.

12. Implant according to one or more of Claims 1 to 11, **characterized in that** the main body has a contour adapted to the shape of an extracted tooth root.

## Revendications

1. Implant, en particulier implant pour racines dentaires, avec un corps de base (1;9;11), réalisé sous la forme de corps en matière synthétique élastique, **caractérisé en ce qu'**au moins une partie de la face extérieure (2) du corps de base (1;9;11) est fibreuse, **en ce que** les fibres (5) de la face extérieure (2) fibreuse, présentent, dans la zone proche du corps de base, chaque fois une zone, de transition (6), ayant une section transversale s'élargissant en allant en vers le corps de base (1;9;11), et **en ce que** la résistance mécanique de la matière synthétique dans la zone des fibres (5) est supérieure à ce qu'elle est dans la zone restante du corps de base (1;9;11).

2. Implant selon la revendication 1, **caractérisé en ce que** le corps de base (9;11) est muni d'un perçage (8) central.

3. Implant selon la revendication 1 ou 2, **caractérisé en ce que** le corps de base (11) est muni de fentes (12) s'étendant sur une partie de sa longueur.

4. Implant selon la revendication 2 ou 3, **caractérisé en ce que** le perçage (8) est muni d'un filetage intérieur (10).

5. Implant selon l'une ou plusieurs des revendications 1 à 4, **caractérisé en ce que** la face extérieure (2) du corps de base est munie de supports d'espacement (14, 15), formés par des saillies du corps de base (Fig. 6).

6. Implant selon la revendication 5, **caractérisé en ce que** les supports d'espacement (14) sont réalisés en forme de mamelons.

7. Implant selon la revendication 5, **caractérisé en ce que** les supports d'espacement (15) sont réalisés en forme de nervures.

8. Implant selon l'une ou plusieurs des revendications 1 à 7, **caractérisé en ce que** la face extérieure (2) du corps de base (11) est munie de nervures longitudinales (13).

9. Implant selon l'une ou plusieurs des revendications 1 à 8, **caractérisé en ce que** la longueur des fibres (5) est inégale.

10. Implant selon l'une ou plusieurs des revendications 1 à 9, **caractérisé en ce que** les fibres (5) présentent une structure de surface diaclasée.

11. Implant selon l'une ou plusieurs des revendications 1 à 10, **caractérisé en ce que** la face extérieure (2) du corps de base (1;9;11) est munie d'un revêtement suscitant la croissance osseuse.

12. Implant selon l'une ou plusieurs des revendications 1 à 11, **caractérisé en ce que** le corps de base présente un contour adapté à la forme d'une racine dentaire extraite.
